(19) Europäisches Patentamt
European Patent Office
Office européen des brevets

(11) Veröffentlichungsnummer : **0 170 099 B1**

(12) **EUROPÄISCHE PATENTSCHRIFT**

(45) Veröffentlichungstag der Patentschrift : **03.01.90**

(51) Int. Cl.[5] : **A 61 K 31/435**

(21) Anmeldenummer : **85108317.0**

(22) Anmeldetag : **05.07.85**

# (54) Verwendung von Lycorin als Immunsuppressor.

(30) Priorität : **16.07.84 DE 3426109**

(43) Veröffentlichungstag der Anmeldung : **05.02.86 Patentblatt 86/06**

(45) Bekanntmachung des Hinweises auf die Patenterteilung : **03.01.90 Patentblatt 90/01**

(84) Benannte Vertragsstaaten : **AT BE CH DE FR GB IT LI LU NL SE**

(56) Entgegenhaltungen : **CHEMICAL ABSTRACTS, Band 80, Seite 18, Nr. 103858s, Columbus, Ohio, US; D.A. ASADOV et al.: "Antiblastic effect of licorine and its derivatives"**

(73) Patentinhaber : **BEHRINGWERKE Aktiengesellschaft Postfach 1140 D-3550 Marburg 1 (DE)**

(72) Erfinder : **Dickneite, Gerhard, Dr. Zum Neuen Hieb 31 D-3550 Marburg-Cappel (DE)**
Erfinder : **Schorlemmer, Hans-Ulrich, Dr. Am Kirschenwald 2 D-3550 Marburg 21 (DE)**
Erfinder : **Sedlacek, Hans-Harald, Dr. Am Sonnenhang 3 D-3550 Marburg (DE)**

(74) Vertreter : **Becker, Heinrich Karl Engelbert, Dr. et al HOECHST AKTIENGESELLSCHAFT Central Patent Department P.O. Box 80 03 20 D-6230 Frankfurt am Main 80 (DE)**

**EP 0 170 099 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung der pharmakologisch wirksamen Substanz Lycorin bzw. ihrer Salze in einem Verfahren zur Herstellung eines Suppressors des Immunsystems bei Säugern.

Für Lycorin ist folgende Struktur beschrieben (Merck Index, 9th Ed.) :

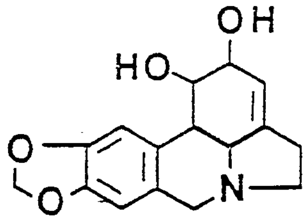

$\cdot HCl \cdot \frac{1}{2} H_2O$

(Lycorin Hydrochlorid-hemihydrat)

Aus Chemical Abstracts 80, 1974, Seite 18, Abstract 103858s, ist bekannt, daß Lycorin eine antiblastische Wirkung besitzt.

Lycorin kann aus Extrakten aus Crinum pratense (Amaryllidaceae) gewonnen werden.

Das Immunsystem der Säuger kann in einen humoralen und einen zellulären Teil gegliedert werden, deren Zusammenwirken für die immunologische Abwehr verantwortlich sind. Die Funktion des Immunsystems besteht in der Elimination von körperfremden Substanzen wie Microorganismen oder Tumorzellen, welche die Funktion eines Organismus beeinträchtigen können. Es gibt jedoch Zustände, die es erforderlich machen, die Immunitätslage eines Organismus im Sinne einer Suppression zu beeinflussen. Ein Beispiel dafür ist der Fall einer immunologischen humoralen und/oder zellulären Reaktion eines Organismus gegen sein körpereigenes Gewebe. Dieses als Autoimmunität oder als Autoaggressivität beschriebene Phänomen kann zu schweren Schädigungen des Organismus führen.

Weitere Beispiele für unerwünschte oder sogar schädigende Wirkung des Immunsystems auf den Organismus sind Immunkomplexerkrankungen, allergische und rheumatische Zustände. Bei der Organtransplantation ist die Unterdrückung der immunologischen Abstoßungsreaktion des Empfängers erforderlich, um das Überleben des transplantierten Organs zu gewährleisten.

In allen oben geschilderten Fällen muß durch geeignete Maßnahmen die Reaktivität des Immunsystems unterdrückt werden. Um dieses zu erreichen, wird mit ionisierenden Strahlen, mit Antikörpern gegen lymphatisches Gewebe oder chemischen Substanzen (chemische Immunsuppressoren) behandelt.

Es wurde nun überraschend gefunden, daß Lycorin Hydrochlorid-hemihydrat die Immunreaktion in Säugern in Mengen von 2,5-20 mg/kg Körpergewicht (in vivo-Reaktionen) sowie die Reaktion von immunologisch kompetenten Zellen, die aus Maus und Mensch gewonnen werden (in vitro-Reaktionen) in Konzentrationen von 0,1-100 µg/ml unterdrücken kann. Die Konzentrationen, die zu einer Immunsuppression führen, lagen deutlich unter den als toxisch erkannten Dosierungen und sind für die Behandlung von Krankheiten, die durch das Immunsystem verursacht wurden, sowie zur Prophylaxe von Transplantatabstoßungen geeignet.

Diese Substanz supprimierte die Aktivität von Maus-Makrophagen in vivo und in der Zellkultur (in vitro) sowie die Funktion von Lymphozyten von Maus und Mensch in der Zellkultur (in vitro).

Gegenstand der Erfindung ist demnach die Verwendung von Lycorin in einem Verfahren zur Herstellung eines Immunsuppressors.

Die wirksame immunsupprimierende Dosierung liegt im Bereich von 2,5-20 mg/kg Körpergewicht. Für die parenterale, speziell intravenöse Verabreichung kommen Lösungen oder Suspensionen des Wirkstoffes in einer pharmazeutisch verträglichen Zubereitung, vorzugsweise gepufferte wässrige Lösungen in Frage.

Im folgenden wird die Einwirkung der Substanz auf die Immunantwort der Maus und des Menschen in ausgewählten in vivo- und in vitro-Standardtestmethoden, die bekanntermaßen für die Beurteilung von Immunsuppressoren herangezogen werden, beispielhaft erläutert.

Beispiel 1

Wirkung von Lycorin auf die Makrophagenaktivität in vitro.

Weibliche NMRI-Mäuse (18-20 g) wurden getötet und die Makrophagen der Bauchhöhle entnommen. Die Makrophagen wurden danach in Petrischalen bei 37 °C in einer 5 ml $CO_2$/100 ml enthaltenden Amosphäre kultiviert. Nach 3 Stunden wurden die schwimmenden Zellen durch Waschen entfernt. Zu den Makrophagen wurde nun Lycorin in Konzentrationen von 50-400 µg/ml hinzugegeben und 24 Stunden stehen gelassen. Der Aktivierungszustand wurde mit Hilfe der Chemolumineszenz gemessen, wobei das Integral Relative Lichteinheiten (RLU) pro 15 Minuten ermittelt wurde. Gemessen wurde sowohl die Wirkung von Lycorin auf die nicht aktivierten (-Zymosan) als auch auf die durch Zymosan aktivierten Makrophagen (+ Zymosan).

Tabelle 1

Wirkung von Lycorin auf die Makrophagen-Chemolumineszenz in vitro

| Lycorin µg/ml Testansatz | Chemolumineszenz RLU / 15 Minuten | |
|---|---|---|
| | - Zymosan | + Zymosan |
| 0 | 701 ± 120 | 5435 ± 233 |
| 50 | 655 ± 75 | 4636 ± 201 |
| 100 | 443 ± 17 | 2510 ± 14 |
| 200 | 219 ± 38 | 1083 ± 166 |
| 400 | 142 ± 39 | 741 ± 167 |

Die Tabelle 1 zeigt, daß die Makrophagenaktivität nach Inkubation mit der Substanz in vitro deutlich reduziert wurde, was sowohl für die unstimulierten (— Zymosan) wie auch die in vitro stimulierten Makrophagen (+ Zymosan) zutrifft.

Beispiel 2

Aktivität von Maus-Makrophagen nach Gabe von Lycorin in vivo.

Weiblichen NMRI-Mäusen (18-20 g) wurde Lycorin Hydrochlorid-hemihydrat in Konzentrationen von 2,5-20 mg/kg intravenös verabreicht. Die Kontrollen erhielten gleiche Volumina des Lösungsmittel (physiologisch gepufferte Kochsalzlösung, pH 7,2). 3 Tage später wurden die Mäuse getötet und ihnen die Makrophagen aus der Bauchhöhle entnommen. Die Makrophagen wurden in Plastikschalen überführt und 3 Stunden bei 37 °C in einer 5 ml $CO_2$/100 ml enthaltenden Atmosphäre kultiviert. Nach Ablauf dieser Zeit wurden die schwimmenden zellen durch Waschen entfernt. Bei einem Teil der Zellen wurde eine Aktivitätsbestimmung mit Hilfe der Chemolumineszene, wie im Beispiel 1 beschrieben, durchgeführt. Der zweite Teil der Zellen wurde weitere 16 Stunden mit der oben beschriebenen Methode kultiviert und nach dieser Zeit die Menge freigesetzter lysosomaler Hydrolasen (N-Acetyl-ß-D-Glucosaminidase) im Überstand gemessen.

Tabelle 2

Aktivität von Maus-Makrophagen nach Gabe von Lycorin in vivo

| Lycorin mg/kg i.v. | Enzymfreisetzung mU/ml N-AcetylGlu | | Chemolumineszenz RLU / 15 Minuten | |
|---|---|---|---|---|
| | - Zymosan | + Zymosan | - Zymosan | + Zymosan |
| 0 | 824 ± 37 | 4786 ± 287 | 2240 ± 358 | 58.900 ± 849 |
| 2,5 | 681 ± 92 | 3748 ± 196 | 1935 ± 35 | 28.750 ± 2331 |
| 5,0 | 473 ± 46 | 2071 ± 149 | 1115 ± 149 | 13.600 ± 2263 |
| 10,0 | 264 ± 57 | 1428 ± 206 | 762 ± 67 | 8.850 ± 1231 |
| 20,0 | 188 ± 18 | 542 ± 82 | 348 ± 39 | 2.105 ± 50 |

Wie aus Tabelle 2 ersichtlich ist, erniedrigt Lycorin die Aktivität von Markophagen, die Mäusen entnommen wurden, welche drei Tage zuvor mit Lycorin behandelt wurden. Diese Suppression wurde sowohl bei der Chemolumineszenz als auch bei der Freisetzung hydrolytischer Enzmye mit und ohne Zusatz von Zymosan beobachtet.

Beispiel 3

Wirkung von Lycorin auf IgM- und IgG-Antikörper-produzierende Milzlymphozyten der Maus in vitro.

Weiblichen NMRI-Mäusen wurden $10^8$ Schafserythrozyten intravenös appliziert. 10 Tage später wurden die Mäuse getötet, die Milzen unter sterilen Bedinungen entnommen, durch ein Sieb gepreßt und so eine Einzelzellsuspension von Milzlymphozyten hergestellt. Die Milzlymphozyten ($8 \times 10^6$/2 ml)

wurden für 4 Tage in Gewebekulturplatten zusammen mit 5 × $10^5$ Schafserythrozyten inkubiert, wobei Lycorin in einer Konzentration von 0.01-1 000 µg/m zugesetzt wurde. Die Kontrolle enthielt kein Lycorin. Die Anzahl der der IgM- und IgG-Antikörper-produzierenden Milzzellen wurde mit Hilfe der bekannten « Plaque forming cell »-Technik (Jerne & Nordin (1963) Science 140, 405) bestimmt.

Die Menge der Plaque forming cells (PFC) gibt die IgM- oder IgG-produzierenden Zellen pro $10^6$ Lymphozyten an.

Tabelle 3

Wirkung von Lycorin auf die IgM- und IgG-Antikörperproduzierenden Milzlymphozyten der Maus

| Lycorin µg/ml Testansatz | Plaque forming cells / $10^6$ Lymphozyten IgM | IgG |
|---|---|---|
| 0 | 957 ± 300 | 221 ± 259 |
| 0,01 | 856 ± 139 | 116 ± 45 |
| 0,1 | 799 ± 335 | 48 ± 24 |
| 1,0 | 5 ± 1 | 9 ± 5 |
| 10,0 | 3 ± 2 | 11 ± 4 |
| 100,0 | 3 ± 1 | 2 ± 2 |

Wie aus Tabelle 3 ersichtlich ist, wurde bei Konzentrationen, die größer als 1,0 µg/ml sind, eine totale Inhibition sowohl bei den IgG- als auch bei den IgM-Antikörper-produzierenden Milzlymphozyten beobachtet. 50 % der Inhibition ($IC_{50}$) wurde bei IgM mit etwa 0,25 µg/ml und bei IgG mit etwa 0,015 µg/ml erzielt.

Beispiel 4

Wirkung von Lycorin auf die Mitogen-stimulierte Proliferation von menschlichen Lymphozyten

Lymphozyten wurden durch Differenzialzentrifugation (Dextran, Ficoll) aus peripherem menschlichem Blut gewonnen. Die Lymphozyten wurden dann für 2 Tage in Microtitrationsplatten (3 × $10^4$ Lymphozyten/200 µl) zusammen mit dem Mitogen Phythämagglutinin (PHA, 5 µg/ml) und Lycorin in Konzentrationen von 0,1-100 µg/ml inkubiert. Danach wurde radioaktives Thymidin ($^{14}C$) zugefügt und weitere 16 Stunden inkubiert. Freies $^{14}C$ Thymidin im Überstand wurde danach im Cell Harvester von den Zellen abgetrennt und die in die Zellen eingebaute Radioaktivität als ein Maß für die Lymphozytenproliferation bestimmt. Als Kontroller wurden Lymphozyten ohne Mitogen und ohne Lycorin mitgeführt. Der Stimulationsindex wurde folgendermaßen ermittelt:

$$\text{Stimulationsindex} = \frac{\text{Radioaktivität Experimentalwert}}{\text{Radioaktivität Kontrollwert}}$$

Erniedrigung des Stimulationsindex bedeutet demnach eine Suppression der Lymphozytenproliferation.

Tabelle 4

Wirkung von Lycorin auf die Mitogen-stimulierte Proliferation menschlicher Lymphozyten in vitro

| Lycorin µg/ml Testansatz | Stimulationsindex |
|---|---|
| 0 | 40 |
| 0,1 | 36 |
| 1,0 | 4 |
| 10,0 | 1 |
| 100,0 | 1 |

Wie aus Tabelle 4 ersichtlich ist, supprimiert Lycorin die Mitogen-stimulierte Proliferation menschlicher Lymphozyten. Totale Suppression wurde bei einer Konzentration von 10 µg/ml Lycorin beobachtet. 50 % Inhibition ($IC_{50}$) wurde mit etwa 0,3 µg/ml erreicht.

Lycorin ist also in in vivo- und in vitro-Testmethoden, die für die Beurteilung von Immunsuppressiva herangezogen werden können, imstande, die immunologische Aktivität des Empfängers zu erniedrigen. Lycorin kann also als Therapeutikum bei Autoimmunerkrankungen, Immunkomplexerkrankungen, allergischen und rheumatischen Zuständen sowie zur Prophylaxe gegen Transplantatabstoßungen angewendet werden.

**Patentansprüche**

1. Verwendung von Lycorin in einem Verfahren zur Herstellung eines Arzneimittels zur Immunsuppression.

2. Verwendung von Lycorin in einem Verfahren zur Herstellung eines Arzneimittels zur Immunsuppression, wobei Lycorin in einer Menge verwendet wird, daß das Arzneimittel 187,5 bis 1 500 Milligramm Lycorin pro Einheitsdosis des Arzneimittels für einen 75 Kilogramm schweren Menschen enthält.

**Claims**

1. The use of lycorine in a process for the preparation of a medicament for immunosuppression.

2. The use of lycorine in a process for the preparation of a medicament for immunosuppression, lycorine being used in an amount such that the medicament contains 187.5 to 1 500 milligrammes of lycorine per unit dose of the medicament for a person weighing 75 kilogrammes.

**Revendications**

1. Utilisation de la lycorine dans un procédé de fabrication d'un médicament immunosuppresseur.

2. Utilisation de la lycorine dans un procédé de fabrication d'un médicament immunosuppresseur, la lycorine étant alors utilisée en quantité telle que le médicament contient de 187,5 à 1 500 milligrammes de lycorine par dose unitaire, pour un homme de 75 kilogrammes.